# EUROPEAN PATENT APPLICATION

(11) **EP 4 667 104 A1**
(43) Date of publication of application: **24.12.2025**
(21) Application number: 24183839.0
(22) Date of filing: 21.06.2024
(51) Int. Cl.: B01L 3/02, C12M 1/26, G01N 35/10

(54) **PIPETTING TIP WITH LATERAL OPENING AND USE OF THE SAME IN ORGANOID SORTING AUTOMATED DEVICES**

(71) Applicant: CSEM Centre Suisse d'Electronique et de Microtechnique SA - Recherche et Développement, 2002 Neuchâtel (CH)
(72) Inventor: GOLDOWSKY, Jonas, 6030 Ebikon (CH); JANDET, Lucie, 6003 Lucerne (CH)
(74) Representative: e-Patent SA

(57) **Abstract**

Pipetting tip for picking and sorting biological entities within a liquid sample having a tubular body defining a pipetting axis, the tubular body comprising a first open end configured for being mounted on a pipetting actuator, and a second open end comprising an opening partially defined on a bottom plane perpendicular to the pipetting axis, said opening further extending on a lateral face of the tubular body such that part of the opening's edge is defined outside said bottom plane.

Method of operating an automated organoid sorting device using the pipetting tip of the invention.

## Description

### Technical Field

The invention is in the field of laboratory pipetting tools.

More specifically, the invention relates to a pipetting tip with a tip opening especially shaped for the handling of large organoids and cell colonies.

The invention also relates to methods of using said pipetting tip in automated organoid handling and sorting devices.

### State of the art

Organoids are three-dimensional structures grown from stem cells that mimic the structure and function of organs. They are typically cultivated in laboratory settings under controlled conditions, allowing researchers to study aspects of organ development, disease progression, and drug responses in a more accurate and ethically acceptable manner compared to traditional animal models or cell cultures. Organoids represent a powerful tool in biomedical research with diverse applications spanning from basic science to clinical translation. The size of an organoid can range from very small structures resembling a few hundred micrometres in diameter to larger, more complex structures that can be several millimetres in size.

Depending on factors like cell source, culture conditions, culture techniques among others, organoids with very heterogeneous properties can result from their maturation in the same culture sample. Many applications, e.g. drug screening, require a selection step, including quality control and individualization (i.e. separation) of the selected organoids before the biological assays can be run.

In general, the handling of organoids with sizes larger than 0.1 mm, particularly more than 0.5 mm and up to 5 mm, is difficult because of their tendency to sediment and their fragility to mechanical stress. While gravity and sedimentation do not play a major role for the handling of small biological entities such as cells, increasing size of such entities means an increasing impact of gravitational forces (∼r³) while the liquid drag force that is used during aspiration is only dependent on the entities cross section (~r²) meaning that the relative velocity of the aspirated liquid surrounding the biological entity must be increased dependent on its size. Unfortunately, most pipetting systems, in particular precision pipetting systems, have a rather limited suction rate which may be unable to appropriately capture a sedimented relatively large biological entity.

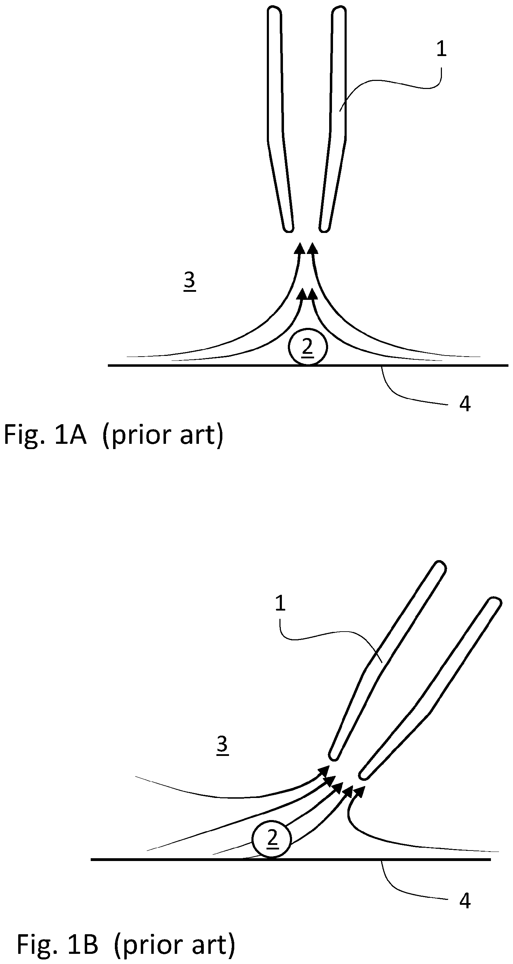

Skilled laboratory technicians know from experience that, in order to capture relatively large biological entities (> 0.5 mm) using a manual pipette, it is convenient to tilt the pipetting tip and approach the biological entity with an angle. Fig. 1 illustrates the advantages of this technique.

Fig. 1A illustrates the standard pipetting procedure with vertical orientation. By vertical orientation it is meant, perpendicular to the bottom of the recipient containing the biological sample. In general, the vertical direction is coincident with the direction of gravity. The pipetting tip 1 approaches from above a target biological entity 2 (which we will for simplicity identify as an "organoid") immersed in a liquid medium 3. Taking into account the boundary effects caused by the bottom of the container 4 containing the sample, only a small portion of the pipette-driven liquid flow leads to an effective force on the organoid 2 directed towards the pipetting tip 1. Only the periphery of the organoid 2 is pulled by the viscous forces of the surrounding liquid 3 and high shear forces are needed to pull the organoid 2 upwards. For valuable and sensitive biological entities such as organoids that are cultured for up to 30 weeks all shear forces need to be reduced to a minimum to preserve their integrity during pipetting steps.

In contrast to the situation of Fig. 1A, an angled suction, as illustrated in Fig. 1B advantageously creates a lateral flow which pushes the target organoid 2 across a larger interaction surface, creating lower shear and requiring lower flow rates to drag the organoid 2 into the pipetting tip 1.

Aware of this manual procedure, some automated organoid handling devices implement tip tilting mechanisms to approach the target entities. For example, in WO2021/019623A1 a cell picking device is disclosed which not only approaches the target cells with a tilted pipetting tip but is further configured to press the tip against the bottom of the cell container in order to elastically bend the tip horizontally to create an efficient lateral flow for dragging the target biological entity.

This approach has several problems. On one hand, the elastic forces involved in bending the tip represent a severe risk for a valuable biological sample, which can be abruptly shattered by an accidental manipulation mistake.

On the other hand, and more generally, approaching the sample with a tilted pipette orientation supposes that the sample container is sufficiently large to geometrically enable this configuration. This can be the case when working for example with samples contained in large Petri dishes. However, there are standard culture vessels, (for example 96 or 384-well plates) where the sample containers have a rather vertical aspect ratio which prevents introducing a pipetting tip sufficiently tilted with respect to the bottom to create the necessary lateral flow. In this case the pipette tilting strategy cannot be applied either manually or even less by an automated organoid handling device.

There is thus a need for a solution allowing a gentle suction of sensitive biological entities into a pipetting tip, which is adapted to access samples contained in deep and narrow sample containers.

### Disclosure of the invention

An aim of the present invention is to propose a pipetting tip allowing a gentle suction of sensitive biological entities, which can at the same time provide access to the bottom of deep and narrow sample containers.

Another aim of the present invention is to propose a method of operation of an automated organoid sorting device using the above-mentioned pipetting tips.

The first aim is achieved by providing a pipetting tip having a tubular body defining a pipetting axis, the tubular body comprising a first open end configured for being mounted on a pipetting actuator, and a second open end comprising an opening surrounded by an opening's edge that is partially located within a bottom plane essentially perpendicular to the pipetting axis. This pipetting tip is in particular recognizable in that said opening further extends on a lateral face of the tubular body such that a lateral part of the opening's edge is defined outside said bottom plane.

Several notions used in the above disclosure are clarified in the following paragraphs.

In this context a "tubular body" refers to an object having cylindrical, quasi-cylindrical or conical shape and a hollow core which can be loaded with a liquid sample. The tubular body has a geometry which is significantly long relative to its diameter. In particular, the tubular body of a pipetting tip is at least 5 times longer than its inner diameter, preferably at least 10 times longer than its inner diameter. By "inner diameter" it is meant the diameter of the hollow core. The hollow core also extends with a cylindrical, quasi-cylindrical or conical shape along the whole length of the tubular body, from the first open end to the second open end. It is understood that the diameter of the "tubular body" may vary along its length, either regularly, as in a purely conical shape, or irregularly. For example, the diameter of the pipetting tip may be constant in a first part, adjacent to the first open end, and later vary, tapering conically towards the second open end. This corresponds to a typical shape of standard pipetting tips.

Regarding the cross section of the tubular body, a circular shape is convenient and generally used, however other shapes could be implemented without departing from the scope of the present disclosure, for example as a regular polygon (e.g. hexagon, octagon) or even other irregular shapes. In the remainder of this document, we will assume for simplicity of the wording that the section of both the hollow core and the external wall of the tubular body is circular. This must be understood to cover the case of irregular shapes as well, wherein the notion of "diameter" or "mean diameter" of the sections should be interpreted as the diameter of the circle which circumscribes the given section.

In this context the "pipetting axis" refers to the axis of the cylindrical, quasi-cylindrical or conical shape defined by the hollow core of the tubular body. While in a preferred embodiment the pipetting axis is a straight line running from the first open end to the second open end of the tubular body (as in the standard straight pipetting tips), it is understood that in some embodiments the tubular body may comprise a curved or bent portion without departing from the scope of the present disclosure. Even in this case, the pipetting axis can be identified as the line running through the geometric centre of the hollow core along the tubular body. When the direction of the "pipetting axis" varies along the length of the pipetting tip, the reference in this text to a direction relative to this axis (e.g. perpendicular or parallel) must be interpreted as relative to the local orientation of the pipetting axis in the concerned part of the tubular body. For example, when referring to a "second open end comprising an opening surrounded by an opening's edge that is partially located within a bottom plane essentially perpendicular to the pipetting axis" it is the direction of the pipetting axis in the extremity of the tubular body corresponding to the second open end that is referred to. In the remainder of this document, we will assume for simplicity that the pipetting axis is a straight line, as in the preferred embodiments. This must be understood to cover the case of curved or bent tubular bodies as well, wherein the direction of the pipetting axis is defined locally, as just explained.

In this context, a "pipetting actuator" refers to a device configured to receive and securely hold a pipetting tip and to control the flow of liquids in or out of the pipetting tip by applying negative or positive pressure inside it.

Standard pipetting tips are coincident with the above definition in that they are generally configured in the form of a plastic tube with a roughly conical shape, the larger end of the cone being configured to be mounted on a pipetting actuator, and the narrower end comprising an opening defining an inlet for a liquid sample. On the other hand, and in contrast to the present invention, the opening at the narrower end of a standard pipetting tip is entirely included within a bottom plane perpendicular to the pipetting axis.

There is a dominant paradigm in the design of pipetting tips which requires that the opening at the narrow end is precisely included within a single plane. Great care is put in manufacturing this opening with an accurate circular and smooth edge, since any irregularity may result in the unintentional dripping of liquid, or in the unwanted trapping of particles or liquid droplets in any irregularity of the opening edges, affecting the volume accuracy of the pipetting operation.

In the approach disclosed in WO2021/019623A1, the inventors go as far as proposing applying a force to literally bend the axis of the pipetting tip, but never deviate from the dominant paradigm of having the tip opening defined within a single plane perpendicular to said axis.

The inventors of the present solution break this paradigm by providing an opening whose edge is not contained in a single plane and is partly defined on the lateral surface of the tubular body of the pipetting tip. As will be explained later, this allows creating a sufficient lateral flow, adapted to drag relatively large sedimented biological entities, while operating the pipetting tip in an essentially vertical position (i.e. perpendicular to the bottom of the recipient containing the biological entity).

Advantageously, the shape of the pipetting tip and in particular of the opening of the second open end can be adapted according to the size of the biological entities which have to be picked and sorted. An optimal flow is created when the size of the opening at the end of the pipette is slightly larger than the maximum diameter of the target biological entity, for example 10% to 100% larger than said maximum diameter.

Indeed, if the opening fits too closely the size of the biological entity, there is an increased risk of damaging it during the pipetting operation. If, on the other hand, the opening is too large, the liquid flow will easily circumvent the target biological entity and the drag force may be insufficient to capture it into the pipetting tip.

In the present disclosure, since the opening is not a simple circular opening contained in a single plane, the notion of "size of the opening" must be clarified. The size of the opening in the present case can be defined according to the orthographic projections of the opening in two main planes: the "bottom plane" and the "lateral plane". The "bottom plane" has already been introduced as the plane perpendicular to the pipetting axis. In the following, the orthographic projection of the opening in the bottom plane will be referred to as the "bottom section" of the opening. In a preferred embodiment (hollow core having cylindric or conical shape), the bottom section of the opening is defined by a circle, and its size simply corresponds to the diameter of this circle.

More generally, the bottom section can advantageously define a quasi-circular shape characterized by a mean diameter.

The "lateral plane" is perpendicular to the bottom plane and thus, parallel to the pipetting axis. The azimuthal orientation of the "lateral plane" is defined as the orientation which maximizes the area of the orthographic projection of the opening in said plane. In other words, the projection of the opening in the "lateral plane" corresponds to the side view of the opening when the pipetting tip is oriented to expose its largest possible lateral window into the hollow core. In the following, the orthographic projection of the opening in the lateral plane will be referred to as the "lateral section" of the opening.

The lateral section of the opening may have different shapes, for example semi-circular or semi-elliptical. Such shape for example corresponds to an opening defined as the geometrical intersection of a cylinder with a plane tilted with respect to its axis. Obviously, the bottom of the lateral section comprises at least a straight segment, coincident with the intersection of the bottom plane and the lateral plane. In an advantageous embodiment, the borders of the lateral section further comprise straight segments parallel to the pipetting axis, and a curved segment corresponding to an arc of ellipse or circle.

In general, the lateral section of the opening can be characterized by two key features which will be simply referred to here as "width" and "height". The "width" corresponds to the maximum distance between the borders of the lateral section, in a direction parallel to the bottom plane. The "height" corresponds to the maximum distance along the direction of the pipetting axis from the bottom plane to the furthest point in the border of the lateral section.

According to an advantageous embodiment, the pipetting tip can be provided with an alignment feature defined at its first open end, which allows defining the orientation of the lateral part of the opening's edge with respect to a pipetting actuator.

The invention further concerns a method of operation of an automated organoid sorting device which takes advantage of these novel pipetting tips. The referred device can be any laboratory equipment configured for picking individual biological entities from a liquid sample, in this case by pipetting, wherein the pipetting operation is controlled by a motorized system, in contrast to manual pipetting, where the pipetting actuator is controlled by the hand of an operating technician. This device will in general comprise a pipetting actuator movable with respect to the sample container. Advantageously, the device may further comprise an identification and localization system, allowing the device, or an operator of the device to precisely localize the target biological entity and to drive the pipetting tip towards it.

The method in question comprises the following steps:
S1) Mounting a pipetting tip as described above on a pipetting actuator of an automated organoid sorting device;
S2) Identifying a target biological entity within a liquid sample, said target biological entity, lying at the bottom of a sample container;
S3) Moving the pipetting tip relative to the sample container to position it such that the bottom plane of the opening is less than 0.5 mm away from the bottom of the sample container, or preferably in contact with it; and the lateral part of the opening's edge is oriented towards the target biological entity, the distance between the biological entity and the pipetting tip being no bigger than twice the maximum diameter of said biological entity, preferably no bigger than the maximum diameter of said biological entity;
S4) Operating the pipetting actuator to suck a defined volume of sample into the pipetting tip, said defined volume of sample comprising the target biological entity; and
S5) Extracting the pipetting tip carrying the target biological entity from the sample container.

The method described above has the advantage that the pipetting tip may be operated in a substantially vertical orientation, i.e. the pipetting axis, or at least the pipetting axis along the portion of the pipetting tip that is introduced in the sample container can be oriented substantially perpendicular to the bottom of the sample container. In this manner, the pipetting tip can access the bottom of deep and narrow sample containers where it would be impossible to introduce a tilted pipetting tip.

In step S3, positioning the bottom plane of the opening close to the bottom of the sample container is meant to restrict the flow of liquid into the pipette through said bottom plane. In this way, the liquid flow into the pipetting tip created in step S4 is mainly a lateral flow which can effectively drag the biological entity.

This restriction of liquid flow from the bottom can in practice be achieved when the distance between the bottom plane of the opening and the bottom of the sample container is much smaller than the height and width of the lateral section; for example, at least 10 times smaller. This means that in practice, in step S3 the bottom of the pipetting tip should be approached to the bottom of the sample container at a distance typically smaller than 0.5 mm, preferably smaller than 0.2 mm.

Preferably a soft contact can be established between the bottom of the pipetting tip and the bottom of the container.

By the design of the pipetting tip, and the way it is mounted in the pipetting actuator, the bottom plane of the opening is advantageously parallel to the bottom of the sample container, such that the latter can effectively block the bottom plane of the opening when these two parallel planes are approached.

As described in step S3, the distance between the target biological entity and the pipetting tip should be no bigger than twice the maximum diameter of said target biological entity. Indeed, if the target biological entity is too far from the opening, the liquid flow at the position of the entity may be too weak to produce the necessary drag force. The referred distance between the target biological entity and the pipetting tip should be interpreted as the distance between the two closest points of these two objects.

Advantageously for the execution of the above method, step S1 may comprise selecting a pipetting tip wherein the height and width of the lateral section of the opening are 10% to 100% larger than the maximum diameter of the target biological entity. As already explained, if the opening fits too closely the size of the biological entity, there is an increased risk of damaging it during the pipetting operation. If, on the other hand, the opening is too large, the liquid flow will easily circumvent the target biological entity and the drag force may be insufficient to capture it into the pipetting tip.

The methods above may comprise further steps aiming at redepositing the target biological entity in a target container. Namely, the methods above may be followed by the steps of:
S6) Moving the pipetting tip relative to a target container to align the second open end of the pipetting tip above a target dispensing position;
S7) Operating the pipetting actuator to release the liquid sample contained in the pipetting tip, at least until the target biological entity carried in said liquid sample is delivered in the target container; and
S8) Extracting the pipetting tip from the target container.

In step S6, the pipetting tip can be advantageously positioned such that the distance between the bottom of the target container and the pipetting tip is in a range between the maximum diameter of the target biological entity and twice this size.

### Brief description of the drawings

Further details of the invention and other advantageous embodiments will appear more clearly upon reading the description below, in connection with the following figures which illustrate:
- Fig. 1: Illustration of different pipetting procedures with standard pipetting tips according to the prior art;
- Fig. 2: Schematic view of different pipet tips according to possible embodiments;
- Fig. 3: Perspective view of a detail of a pipetting tip according to one embodiment;
- Fig. 4: Schematic lateral view of a detail of a pipetting tip according to the embodiment of Fig. 3;
- Fig. 5: Perspective view of another detail of a pipetting tip according to one embodiment;
- Fig. 6: Block diagram describing an exemplary method for sorting biological entities according to one aspect of the invention, and
- Fig. 7: Illustration of different steps of exemplary methods for sorting biological entities according to one aspect of the invention.

### Embodiments of the invention

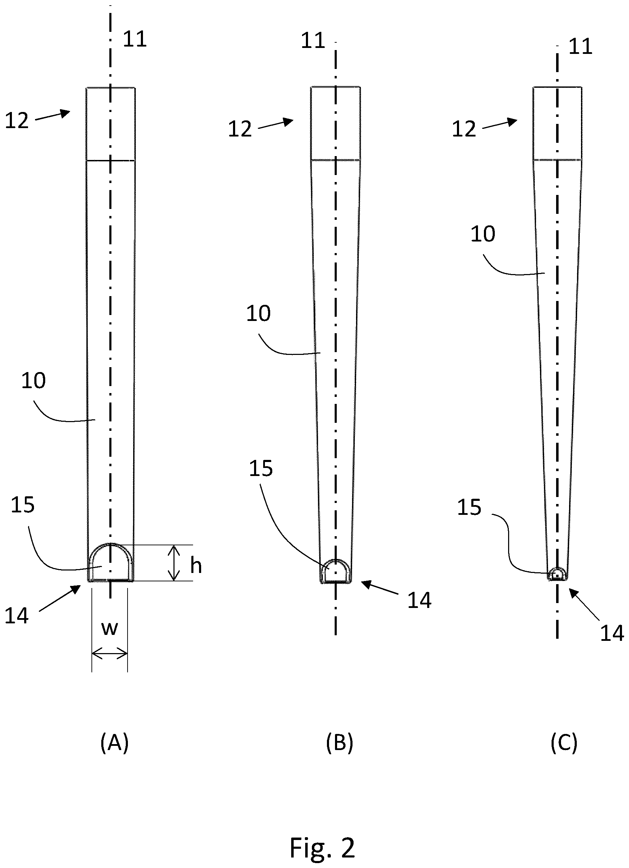

Fig. 2 presents a schematic view of different pipetting tips 10 according to possible embodiments. The views are represented as an orthographic projection of the pipetting tips on their "lateral plane", as defined before.

In all the represented cases, the tubular body of the pipetting tip presents a straight geometry defining a straight pipetting axis 11. A first open end 12 is configured for being mounted on a pipetting actuator, whereas the second open end 14 comprises an opening 15 according to possible embodiments of the invention.

In the represented embodiments, the open ends 12 of all three pipetting tips 10 are identical, such that any of them can be mounted on the same pipetting actuator.

The form of the pipetting tips 10, in particular towards the second open end 14 differs in these exemplary embodiments, as each pipetting tip is adapted to handle biological entities of different sizes.

The first pipetting tip 10 (Fig. 2A) has a cylindrical shape (constant diameter along its entire length). The hollow core of this tip also has a cylindrical shape, with an inner diameter of 5 mm. It is thus well adapted to handle large biological entities with characteristic size of several millimetres.

In these views, the opening 15 at the second end 14 is seen as projected on the lateral plane. Therefore, only the lateral section of the opening is visible in this perspective.

The second and third pipetting tips 10 (Fig. 2 B, C) present a conical shape, tapering towards the second open end 14. For example, the section of the pipetting tip of Fig. 2B at the second open end has an inner diameter of 3 mm, while the pipetting tip of Fig. 2C has an inner diameter of 1.5 mm at its second open end. The conical shape advantageously allows adapting the size of the opening 15 at the second open end 14 for different sizes of biological entities, while keeping a standard mounting interface at the first open end 12.

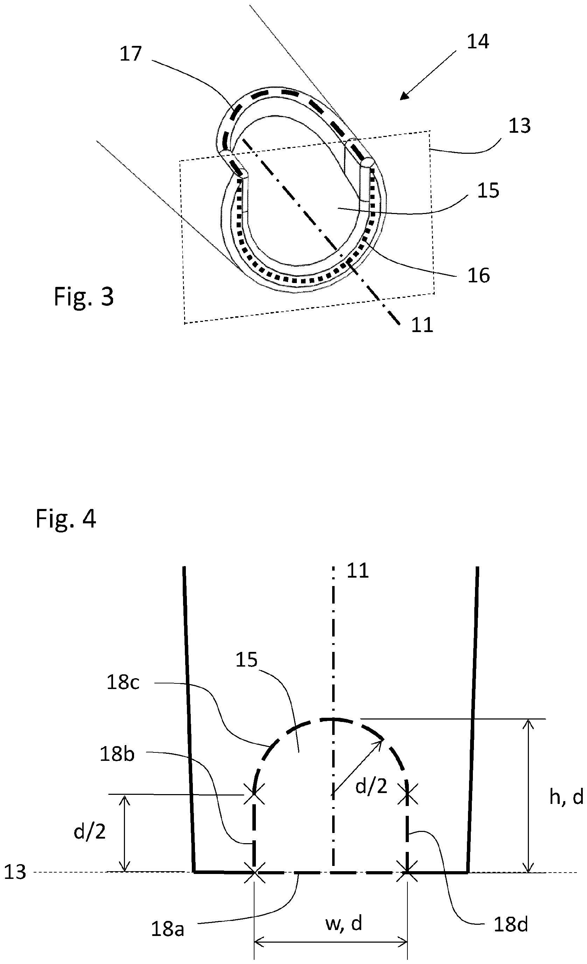

Fig. 3 presents a perspective view of the second open end 14 of a pipetting tip according to one embodiment. In this perspective view, the whole perimeter of the opening 15 can be seen. A first part 16 of the opening's perimeter or edge is defined within a bottom plane 13, perpendicular to the pipetting axis 11. A second lateral part 17 of the opening's perimeter is defined on a lateral face of the pipetting tip, out of said bottom plane 13. This second lateral part 17 in the present example is not contained in a single plane.

The first part 16 of the opening's perimeter, defined on the bottom plane 13, can advantageously contact the bottom of a sample container to hinder the flow of liquid between the pipetting tip and the container across said bottom plane 13. When the bottom section of the opening 15 is obstructed by a container, the liquid flow is driven laterally (i.e. tangential to the bottom of the container) across the lateral section of the opening 15.

Fig. 4 shows a schematic lateral view of the opening 15 of Fig. 3. The view is represented as an orthographic projection of the pipetting tip 10 on its "lateral plane", as defined before. The discontinuous line represents thus the borders 18a, 18b, 18c, 18d of the "lateral section" of the opening. This discontinuous line is the orthographic projection of the opening's perimeter 16, 17 on the lateral plane. The width w and the height h of the lateral section as defined before are represented.

According to an advantageous embodiment, the inventors have realized that an optimal opening for handling biological entities with these pipetting tips present a height h similar to the mean diameter d of the bottom section of the opening. By "similar" it is meant, within a range of plus or minus 20% of said bottom section mean diameter. In other words, the height h of the lateral section of the opening 15 is in a range from 80% to 120% of the mean diameter d of the bottom section of the opening, preferably in a range from 90% to 110% of the mean diameter of the bottom section.

In the example of Fig. 4, the height h of the lateral section of the opening 15 is equal to the diameter d of the arc of circle defined by the opening's edge 16 coincident with the bottom plane 13.

Similarly, according to an advantageous embodiment, the inventors have realized that an optimal opening for handling biological entities with these pipetting tips present a width w similar to the mean diameter d of the bottom section of the opening. By "similar" it is meant, within a range of plus or minus 20% of said bottom section mean diameter. In other words, the width w of the lateral section of the opening 15 is in a range from 80% to 120% of the mean diameter d of the bottom section of the opening, preferably in a range from 90% to 110% of the mean diameter of the bottom section.

In the example of Fig. 4, the width w of the lateral section of the opening 15 is equal to the diameter d of the arc of circle defined by the opening's edge 16 coincident with the bottom plane 13.

The lateral section of the opening 15 represented in Fig. 4 has an advantageous geometry defined by the borders 18a, 18b, 18c, 18d, wherein said borders comprise a first straight segment 18a, parallel to the bottom plane 13 and having a length corresponding to the diameter of the bottom section d. Said first straight segment 18a is connected to a second straight segment 18b, parallel to the pipetting axis 11 and having a length corresponding to half of said diameter d. Said second straight segment 18b is further connected to a curvilinear segment 18c in the form of an arc of circle culminating at a height h with respect to the bottom plane equal to said diameter d. Said arc of circle 18c, in the example of Fig. 4, covers an angular range of 180°and has a radius corresponding to half of the diameter d of the bottom section. The curvilinear segment 18c is further connected to a third straight segment 18d parallel to the pipetting axis 11 and having a length corresponding to half of the diameter d of the bottom section. Said third straight segment 18d is further connected to the said first straight segment 18a, thus completing the closed perimeter of the lateral section of the opening 15.

This geometry of the lateral section is advantageous in that it procures a good matching of the opening's shape with the form of biological entities (typically ellipsoids). Also, it avoids sharp angles which could trap particles or mechanically affect the biological entities. At the same time, its simple design enables large scale fabrication, for example, by injection molding.

According to possible embodiments, the pipetting tip may comprise a thermoplastic material, preferably a polycarbonate (PC), polypropylene (PP), polystyrene (PS), cyclic olefin polymer (COP), or cyclic olefin copolymer (COC). All these materials are advantageous in that they are compatible with biological applications and suitable for large scale fabrication, for example, by injection molding.

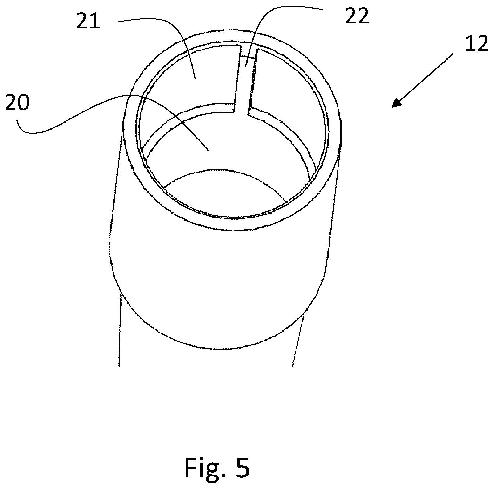

Fig. 5 presents a perspective view of a detail of the first open end 12 of a pipetting tip according to one embodiment. The first open end 12 is configured for being mounted on a pipetting actuator (not represented). In this example, a mounting surface 20 of the inner surface of the pipetting tip is configured to match an external mounting surface of the pipetting actuator, providing an air-tight fixation interface. Air tightness is necessary to allow the pipetting actuation through positive and negative pressures applied inside the pipetting tip.

Advantageously, in the embodiment of Fig. 5 a recess 21 provided in the mounting surface 20 defines an alignment feature 22, which has a known orientation with respect to the lateral part of the opening's edge 17 in the second open end 14 (not represented in Fig. 5) of the pipetting tip. Correspondingly, a pipetting actuator designed for this pipetting tip can advantageously present a protrusion on its external mounting surface designed to match the recess 21 and the alignment feature 22. In this way, when the pipetting tip is mounted on the pipetting actuator, the orientation of the lateral part of the opening's edge 17 of the pipetting tip is exactly defined with respect to the pipetting actuator. This is particularly advantageous when the pipetting actuator is part of an automated organoid sorting device.

As the skilled person would rapidly recognize, an alignment feature can also be implemented in other manners beyond the example of Fig. 5. For example, the alignment feature may comprise a plurality of recesses defined on the inner surface of the pipetting tip. The alignment feature may also be defined on the external surface of the pipetting tip, or it may even consist of an opening in the border of the first open end 12 of the pipetting tip, configured to match with a protruding element of the pipetting actuator mounting surface.

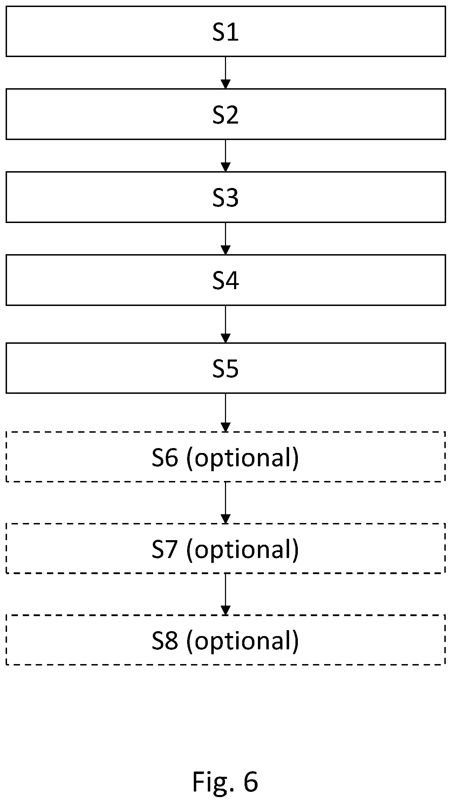

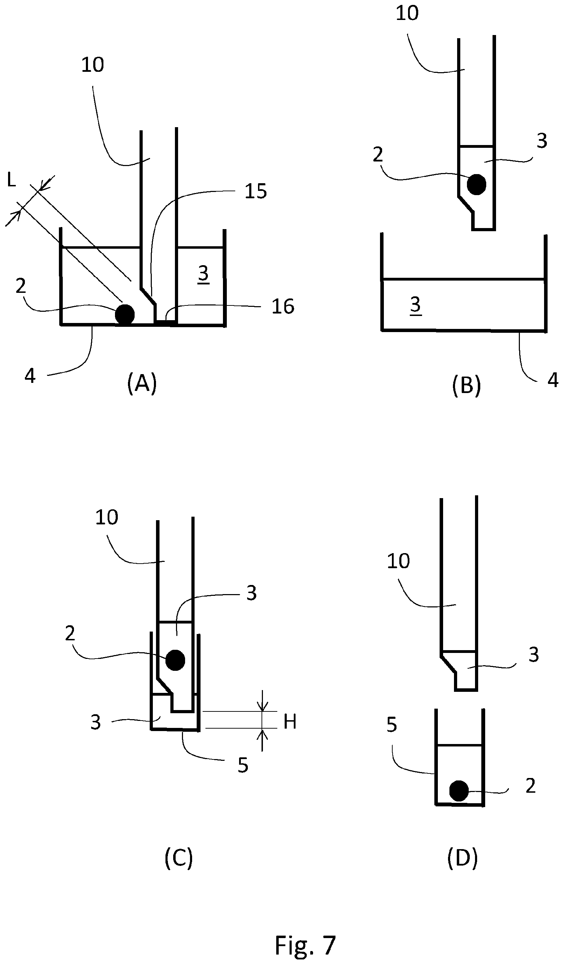

Fig. 6 presents a block diagram describing an exemplary method for sorting biological entities with an automated organoid sorting device according to one aspect of the invention. Fig. 7 illustrates some of the steps of said method.

According to the embodiment illustrated in Fig. 6, the method comprises the sequence of steps S1 to S5, and optional additional steps S6 to S8 as will be described in the following paragraphs.

The step S1 comprises mounting a pipetting tip according to the above disclosed first aspect of the invention on a pipetting actuator of an automated organoid sorting device. Advantageously, the mounted pipetting tip can be held by the pipetting actuator in a substantially vertical position.

The step S1 can be manually performed by a laboratory technician or be automated. For example, the pipetting actuator can be mounted on a movable arm or carrier which can access a supply box of pipetting tips and mount one of them on the actuator by introducing it in the first open end of the tip and applying a predetermined pressure to procure the air-tight fixation.

The step S2 comprises identifying a target biological entity within a liquid sample, said target biological entity, lying at the bottom of a sample container. This identification may be totally automated, for example, if the automated organoid sorting device comprises a smart machine vision system trained to recognize specific target biological entities. Alternatively, the identification of the target biological entity may be performed by an operator who indicates the position of the target entity in a visual interface of the device.

The step S3 comprises moving the pipetting tip relative to the sample container to position it such that the bottom section of the opening is close to the bottom of the sample container, preferably in contact with it; and the lateral section of the opening is oriented towards the target biological entity. The distance between the biological entity and the pipetting tip should be no bigger than twice the maximum diameter of said biological entity.

The relative motion of the pipetting tip with respect to the sample can be implemented either by moving the pipetting actuator carrying the tip relative to a fixed laboratory frame, or by moving the sample container relative to a fixed laboratory frame, or a combination of both.

For example, the sample container may be mounted on a motorized X-Y stage, while the pipetting actuator may be configured to displace the tip along a vertical axis only. Alternatively, the sample container may be fixed to the device, unmovable relative to a fixed laboratory frame, and the pipetting actuator may be mounted in an X-Y-Z carrier.

The logics of the motion of the pipetting tip with respect to the sample can be arranged in different manners according to different device designs. In general, it will be advantageous to perform as final motion, the vertical approach of the pipetting tip close to the bottom of the sample container, once the position of the tip in the X-Y (horizontal) plane has been set. Indeed, once the tip has contacted the bottom of the container, further motion along the horizontal plane can be restricted by the friction forces. A stick-slip type of displacement of the tip against the bottom of the container should be avoided since it could easily result in damaging the target entities.

In some embodiments, the final vertical approach of the pipetting tip towards the bottom of the sample container may be controlled by contact sensors integrated in the automated organoid sorting device. For example, the pipetting actuator or a platform where the sample container is mounted may comprise a pressure sensor to detect the moment when the approaching pipetting tip contacts the bottom of the sample container. In other cases, the detection of contact between the pipetting tip and the bottom of the sample container may be realized by optical means. For example, the illumination of the bottom edge of the opening as seen from a camera observing the bottom of the container can sensibly change when contact is established between said edge and the container.

In some automated organoid sorting devices, the orientation of the lateral section of the opening may be adjusted, for example by using a pipetting actuator provided with a rotational degree of freedom. Such implementation has the advantage that a target entity can be approached from any side since there is the possibility of orienting the lateral section of the opening always towards the target entity. The side from which the target entity is approached can be decided according to different criteria. For example, the target entity should be approached from a side free of other entities which should not be picked up with the target organoid. Also, the target entity should be approached from a side where there is no collision between the tip and the walls of the sample container or any other obstacle.

In the case of automated organoid sorting devices which do not have the possibility of orienting the pipetting tip, the target entity should be approached from a predetermined side according to the orientation of the mounted tip.

In all cases the step S3 is substantially simplified by the presence of an alignment feature in the pipetting tip as described before. Alternatively, if the pipetting tip was mounted on the pipetting actuator without any determined orientation, it would be still possible to implement the step S3 by identifying the orientation of the tip through visual inspection. Once the orientation is known, the position of the tip for the approach of step S3 can be determined. All of this can be done in a fully automated manner for example by implementing a trained machine vision algorithm, or with the partial intervention of an operator.

Fig. 7A schematically illustrates the situation after completion of the step S3 of the method according to an embodiment. The pipetting tip 10 is positioned with the edge 16 of the bottom section of the opening 15 in close contact with the bottom of a sample container 4. A target biological entity 2 of roughly spherical shape in this example, is immersed in a liquid 3 and lies at the bottom of the sample container 4. The lateral section is directly facing in the direction of the target entity 2 (the opening 15 is not represented frontally as in Fig. 4, but from the side). Conveniently, the distance L between the pipetting tip 10 and the target biological entity 2 is similar to the diameter of said target entity 2.

After completing the positioning of the pipetting tip as described in step S3 and illustrated in Fig. 7A, the next step S4 comprises operating the pipetting actuator to suck a defined volume of sample into the pipetting tip, said defined volume of sample comprising the target biological entity.

Once the target entity 2 has been picked into the pipetting tip 10, a further step S5 comprises extracting the pipetting tip 10 from the sample container, as represented in Fig. 7B.

In this Fig. 7B, the level of liquid 3 remaining in the container 4 is depicted as lower than the liquid level of Fig. 3A. This is meant to represent the fact that the volume of the pipetting tip itself has been extracted from the container in Fig. 7B, but also that, part of the liquid 3 originally contained in the container 4 has been removed together with the target entity 2. When there are several target entities to extract from a container, it can be convenient to provide the automated organoid sorting device with a liquid refilling system which injects in the sample container 4 the amount of liquid corresponding to the defined volume of sample sucked into the pipetting tip in step S4. This refilling of liquid could be performed either simultaneously with the aspiration of liquid in step S4, after or even before this operation, according to the design of the container and the automated organoid sorting device.

It must be noted that in both steps S3 and S5 the relative motion of the pipetting tip 10 into and out of the container 4 can be performed along a substantially vertical direction, allowing access to the bottom of relatively narrow and deep sample containers. The X-Y positioning of the pipetting tip 10 with respect to the sample container 4 can advantageously be performed when the pipetting tip is above and outside the container inner volume.

The exemplary method illustrated in Fig. 6 can be advantageously completed by additional steps S6 to S8 aiming at transferring the picked target biological entity 2 to a target container 5.

According to an embodiment, the step S6 comprises moving the pipetting tip relative to a target container to align the second open end of the pipetting tip above a target dispensing position within said target container.

The relative motion of the pipetting tip with respect to the target container 5 can be implemented either by moving the pipetting actuator carrying the tip relative to a fixed laboratory frame, or by moving the target container 5 relative to a fixed laboratory frame, or a combination of both.

The target container 5 is often a separate container where further tests or maturation can be performed on a selected biological entity 2. On the other hand, in some cases, the target container 5 could be the same sample container 4 referred in steps S1 to S5. Indeed, one possible application of an organoid sorting device can be to reposition within a sample container 4, different biological entities, for example to group them or to induce certain specific interactions between them. Therefore, step S6 comprises positioning the pipetting tip not just above a target container, but specifically above a target dispensing position therein. Of course, in many organoid separation routines, there is no special need for dispensing the selected organoids at a particular place within the target containers. In this case the target dispensing position may be chosen anywhere within the target container, for example at its centre.

In step S6, the vertical distance between the pipetting tip and the bottom of the target container can be advantageously adjusted depending on the specific characteristics of the device, the containers and the biological samples which are handled.

For example, in Fig. 7C the target container 5 is represented as a narrow well barely larger than the pipetting tip 10. In this case, if the pipetting tip 10 was positioned in contact or at a short distance from the bottom of the target container 5, the dispensing of the biological entity 2 would be hindered by the neighbouring container walls. It is thus advisable to respect a certain vertical distance H between the bottom of the target container 5 and the pipetting tip 10.

The distance H between the bottom of the target container 5 and the pipetting tip 10 can advantageously be in a range between the maximum diameter of the biological entity 2 and twice this size. A shorter vertical distance H would result later in a predominantly lateral flow when the entity 2 is pushed out of the pipetting tip. In a narrow well, as represented in Fig. 7C this can result in a turbulent lateral flow against the neighbouring walls which could potentially damage the biological entity 2. Even in a larger container, a dominant lateral flow for the dispensing of the selected entity 2 is inconvenient because it reduces the precision of the final position of the target entity with respect to the target dispensing position.

Similarly, a too long vertical distance H between the bottom of the target container 5 and the pipetting tip can result in an unprecise "sinking" of the target entity 2 within the liquid medium 3, with a consequent loss of positioning precision.

Fig. 7C depicts a target container 5 which is partially prefilled with a liquid medium 3. Advantageously, the pipetting tip is already in contact with said prefilled liquid 3 at the end of step S6. This allows a controlled laminar flow of the liquid that will be extracted from the pipetting tip in the next step S7, which is convenient for the positioning precision of the selected entity 2.

On the other hand, it is not mandatory that the target container contains already any liquid. In many cases the container may be empty, and the culture liquid is simply provided with the selected entity 2 from the content of the pipetting tip. Also, the precision of the dispensing is not a major concern in many applications.

It is however advantageous to position the pipetting tip such that the liquid which will be dispensed does not break down as separate droplets when the liquid is ejected. Breaking droplets not only result in a poor positioning precision, but also produce shock waves within the liquid medium which can damage the target biological entities. Therefore, in step S6, it is advantageous to position the opening of the pipetting tip either in contact with a liquid medium which prefills the target container 5, or at least at a distance from the bottom or from a container wall, or from the surface of a liquid prefilling said container, sufficiently small to establish contact with a forming droplet at the opening of the tip before said droplet grows enough to break apart into a free fall.

After completing the step S6, a step S7 of the examplary method of Fig. 6 comprises operating the pipetting actuator to release the liquid sample contained in the pipetting tip, at least until the target biological entity 2 is delivered in the target container.

In some cases, the whole liquid content of the pipetting tip can be dispensed with the target biological entity 2. In other cases, it may be advantageous to limit the dispensed volume of liquid. Therefore, the step S7 may be interrupted as soon as the target entity 2 is released from the tip.

As discussed above, for the sake of the positioning precision and in order to preserve the biological entity 2 from damaging mechanical shakes, it is convenient to release the liquid slowly, such as to create a controlled laminar flow.

A possible intermediary step of the method (not represented in Fig. 6) may be introduced between the steps S5 and S7, prior or after the step S6. This additional intermediary step would comprise waiting a sufficient time, typically a few seconds but possibly up to a few minutes, until the target biological entity trapped in the pipetting tip sinks onto the bottom of the liquid volume trapped in the pipetting tip.

One advantage of this optional intermediary step would be that, in the dispensing step S7, the target biological entity will be promptly released from the pipetting tip, with minimum dispensing of the surrounding trapped liquid. This can be interesting for example when the biological entity must be transferred to a different culture medium with specific product concentrations which should not be altered by a massive apport of the original culture medium from the sample container.

The final step S8 of the embodiment of Fig. 6 comprises extracting the pipetting tip from the target container. The pipetting tip may still contain a certain volume of the liquid previously extracted from the sample container. The final situation, after completing the transfer of the target biological entity is illustrated in Fig. 7D.

A rapid vertical extraction of the pipetting tip in the step S8 can advantageously contribute to limiting the diffusion of the liquid previously extracted from the sample container into the target container.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims.

## Claims

1. Pipetting tip (10) for picking and sorting biological entities (2) within a liquid sample (3), said biological entities (2) having a characteristic size between 0.1 mm and 5 mm, preferably between 0.5 mm and 5 mm, the pipetting tip (10) having a tubular body defining a pipetting axis (11), the tubular body comprising a first open end (12) configured for being mounted on a pipetting actuator, and a second open end (14) comprising an opening (15) surrounded by an opening's edge (16, 17) that is partially located within a bottom plane (13) essentially perpendicular to the pipetting axis (11), **characterized in that** said opening (15) further extends on a lateral face of the tubular body such that a lateral part (17) of the opening's edge is defined outside said bottom plane (13).

2. Pipetting tip according to claim 1, wherein the orthographic projection of the opening's edge (16, 17) onto the bottom plane (13) defines a quasi-circle, preferably a circle, **characterized by** a mean diameter (d).

3. Pipetting tip (10) according to claim 2, wherein the maximum distance (h) between said lateral part (17) of the opening's edge and said bottom plane (13), is in a range from 80% to 120% of said mean diameter (d), preferably in a range from 90% to 110% of said mean diameter (d).

4. Pipetting tip (10) according to claim 2, wherein the maximum distance (w) between two points of said lateral part (17) of the opening's edge, measured parallel to said bottom plane (13), is in a range from 80% to 120% of said mean diameter (d), preferably in a range from 90% to 110% of said mean diameter (d).

5. Pipetting tip (10) according to claims 3 and 4, wherein the orthographic projection of the opening (15) onto a lateral plane perpendicular to the bottom plane (13) and oriented such as to maximize the area of said orthographic projection of the opening (15) onto said lateral plane, defines a lateral section delimited by a perimeter (18a, 18b, 18c, 18d) comprising a first straight segment (18a), parallel to the bottom plane (13) and having a length (w) corresponding to said mean diameter (d), said first straight segment (18a) being connected to a second straight segment (18b), parallel to the pipetting axis (11) and having a length corresponding to half of said mean diameter (d), said second straight segment (18b) being further connected to a curvilinear segment (18c) in the form of an arc of circle or ellipse culminating at a height (h) with respect to the bottom plane (13) corresponding to said mean diameter (d), said curvilinear segment (18c) being further connected to a third straight segment (18d) parallel to the pipetting axis (11) and having a length corresponding to the half of said mean diameter (d), wherein said third straight segment (18d) is further connected to said first straight segment (18a).

6. Pipetting tip (10) according to claim 5, wherein said curvilinear segment (18c) corresponds to an arc of a circle of diameter equal to said mean diameter (d).

7. Pipetting tip (10) according to any of the preceding claims comprising a thermoplastic material, preferably a polycarbonate (PC), polypropylene (PP), polystyrene (PS), cyclic olefin polymer (COP), or cyclic olefin copolymer (COC).

8. Pipetting tip (10) according to any of the preceding claims, wherein the first open end (12) comprises an alignment feature (22) defining the orientation of the opening (15) around the pipetting axis (11), with respect to a pipetting actuator configured to cooperate with said alignment feature (22).

9. Method of operating an automated organoid sorting device comprising the steps of:
S1) Mounting a pipetting tip (10) according to any of claims 1 to 8 on a pipetting actuator of the automated organoid sorting device;
S2) Identifying a target biological entity (2) within a liquid sample (3), said target biological entity (2) lying at the bottom of a sample container (4);
S3) Moving the pipetting tip (10) relative to the sample container (4) to position it such that the bottom plane (13) of the opening (15) is less than 0.5 mm away from the bottom of the sample container (4), or preferably in contact with it, and such that the lateral face of the tubular body comprising the lateral part (17) of the opening's edge is oriented towards the target biological entity (2), the distance between the target biological entity (2) and the pipetting tip being no bigger than twice the maximum diameter of said target biological entity (2), preferably no bigger than the maximum diameter of said target biological entity (2);
S4) Operating the pipetting actuator to suck a defined volume of sample into the pipetting tip (10), said defined volume of sample comprising the target biological entity (2); and
S5) Extracting the pipetting tip (10) carrying the target biological entity (2) from the sample container (4).

10. Method according to claim 9, wherein step S3 comprises a final phase of moving the pipetting tip (10) relative to the sample container (4) in a direction perpendicular to the bottom of said sample container (4).

11. Method according to claim 10, wherein said final phase is controlled by contact sensors integrated in the automated organoid sorting device.

12. Method according to any of claims 9 to 11, wherein the step S3 comprises a phase of orienting the pipetting tip (10) by a rotational motion of the pipetting actuator around the pipetting axis (11).

13. Method according to any of claims 9 to 12, wherein the step S1 comprises selecting a pipetting tip (10) having an opening (15) with characteristic sizes of width (w) and height (h) of the lateral part (17) of the opening's edge, which are 10% to 100% larger than the maximum diameter of the target biological entity (2).

14. Method according to any of claims 9 or 13, further comprising the steps of
S6) Moving the pipetting tip (10) relative to a target container (5) to align the second open end (14) of the pipetting tip (10) above a target dispensing position within said target container (5);
S7) Operating the pipetting actuator to release the liquid sample contained in the pipetting tip (10), at least until the target biological entity (2) carried in said liquid sample is delivered in the target container (5); and
S8) Extracting the pipetting tip (10) from the target container (5).

15. Method according to claim 14, wherein step S6 comprises positioning the pipetting tip (10) above the target dispensing position at a distance from the bottom of the target container (5) being in a range between the maximum diameter of the target biological entity (2) and twice said maximum diameter.
